(19) Europäisches Patentamt
European Patent Office
Office européen des brevets

(11) EP 1 292 557 B1

(12) # FASCICULE DE BREVET EUROPEEN

(45) Date de publication et mention de la délivrance du brevet:
**23.08.2006 Bulletin 2006/34**

(21) Numéro de dépôt: **01947589.6**

(22) Date de dépôt: **22.06.2001**

(51) Int Cl.:
***C07C 41/09*** *(2006.01)* ***C07C 43/13*** *(2006.01)*
***B01J 29/00*** *(2006.01)*

(86) Numéro de dépôt international:
**PCT/FR2001/001980**

(87) Numéro de publication internationale:
**WO 2001/098243 (27.12.2001 Gazette 2001/52)**

(54) **PROCEDES D'ETHERIFICATION DU GLYCEROL, ET CATALYSEURS POUR LA MISE EN OEUVRE DE CES PROCEDES**

VERFAHREN UND KATALYSATOREN ZUR ETHERIFIZIERUNG VON GLYCEROL

METHODS FOR ETHERIFYING GLYCEROL, AND CATALYSTS FOR IMPLEMENTING SAID METHODS

(84) Etats contractants désignés:
**AT BE CH CY DE DK ES FI FR GB GR IE IT LI LU MC NL PT SE TR**

(30) Priorité: **23.06.2000 FR 0008078**

(43) Date de publication de la demande:
**19.03.2003 Bulletin 2003/12**

(73) Titulaire: **CENTRE NATIONAL DE LA RECHERCHE SCIENTIFIQUE (CNRS)
75794 Paris Cedex 16 (FR)**

(72) Inventeurs:
- **BARRAULT,Joel
F-86240 Liguge (FR)**
- **CLACENS, Jean-Marc
F-69006 Lyon (FR)**
- **POUILLOUX, Yannick
F-86550 Mignaloux (FR)**

(74) Mandataire: **Grosset-Fournier, Chantal Catherine et al
Grosset-Fournier & Demachy,
54, rue Saint-Lazare
75009 Paris (FR)**

(56) Documents cités:
**US-A- 5 349 094** **US-A- 5 635 588**

- **J-M CLACENS: "Mesoporous basic catalysts: comparison with alkaline exchange zeolites (basicity and porosity). Application to the selective etherification of glycerol to polyglycerol" STUDIES IN SURFACE SCIENCE AND CATALYSIS., vol. 118, 1998, pages 895-899, XP000992504 ELSEVIER SCIENCE B.V., AMSTERDAM., NL ISSN: 0167-2991 cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 292 557 B1

## Description

La présente invention a pour objet de nouveaux procédés d'éthérification par polymérisation du glycérol ou du glycidol, principalement en vue de l'obtention de compositions contenant essentiellement du diglycérol et/ou du triglycérol, ainsi que de nouveaux catalyseurs pour la mise en oeuvre de ces procédés.

Depuis de nombreuses années, l'industrie chimique puise dans les ressources de carbone fossile (pétrole, gaz naturel, charbon) l'essentiel des matières premières indispensables. Cependant, en raison de l'épuisement inévitable de ces gisements et de normes environnementales de plus en plus strictes, un intérêt croissant est porté sur les matières premières d'origine végétale en raison de leur caractère renouvelable et évolutif. En effet, l'utilisation des huiles et corps gras naturels semble maintenant possible dans l'industrie de la chimie des détergents ou de la lubrification, pour l'obtention des composés issus jusqu'alors de la pétrochimie. Néanmoins, la transformation d'huiles végétales entraîne la formation de co-produits, comme le glycérol, qu'il faut valoriser (le terme de glycérol s'applique au produit pur, par contre, le terme glycérine est utilisé pour le produit commercial contenant 95 % de glycérol).

Parmi les réactions intéressantes, l'éthérification constitue une des voies de valorisation du glycérol en vue de l'obtention d'éthers de glycérols, encore désignés polyglycérols. Les éthers de glycérol ont différentes applications en fonction de leur préparation et de leur structure. Mais, leur principale utilisation se situe dans la préparation des lubrifiants. Les éthers de glycérol trouvent aussi des applications comme tensio-actifs neutres, dans la dépollution des sols, dans l'industrie de la cosmétique, de la pharmacie, dans l'industrie agro-alimentaire. Les polyglycérols sont essentiellement des intermédiaires de synthèse des esters de polyglycérols qui sont utilisés comme tensio-actifs non ioniques et comme substituts des triglycérides dans l'alimentation humaine (composés à faible teneur calorique).

Ainsi, les éthers de glycérol, peu étudiés jusqu'à présent, se révèlent avoir des atouts non négligeables. Le développement d'un mode de préparation sélectif de polyglycérols, tels que le di et/ou le triglycérol, ferait de ces produits des composés intéressants sur le marché oléochimique.

Les polyglycérols sont généralement obtenus par réaction du glycérol avec lui-même pour conduire à des éthers de glycérol suite à une réaction de déshydratation intermoléculaire selon le schéma suivant :

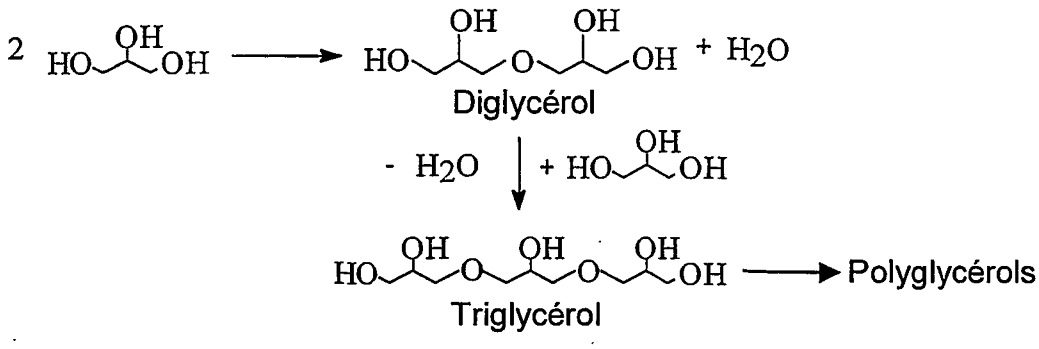


Au vu de la littérature, il apparaît que la réaction de formation sélective du diglycérol et du triglycérol est un processus complexe. En outre, lorsque les fonctions alcools primaires (position $\alpha$) sont impliquées, la réaction conduit à des polymères linéaires. Mais, une déshydratation intramoléculaire des éthers de polyglycérol peut être observée, conduisant à la formation d'époxy-éthers et de composés cycliques de formules suivantes :

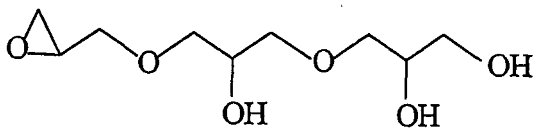

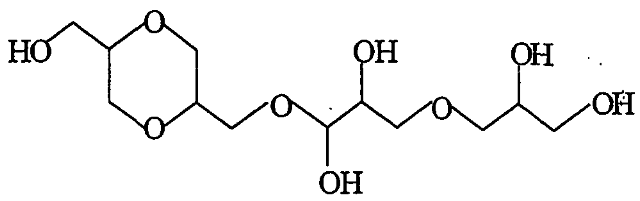

Cette réaction de polymérisation est généralement réalisée à pression atmosphérique ou sous pression réduite, afin d'extraire en continu et plus rapidement l'eau formée. De plus, il est préconisé de ne pas travailler à des températures supérieures à 270°C, car le glycérol est instable à cette température.

Il est recommandé de réaliser l'expérience sous atmosphère de gaz inerte ($CO_2$, $N_2$) afin d'éviter la déshydratation du glycérol pouvant conduire à la formation de produits secondaires indésirables tels que l'acroléine selon le schéma suivant :

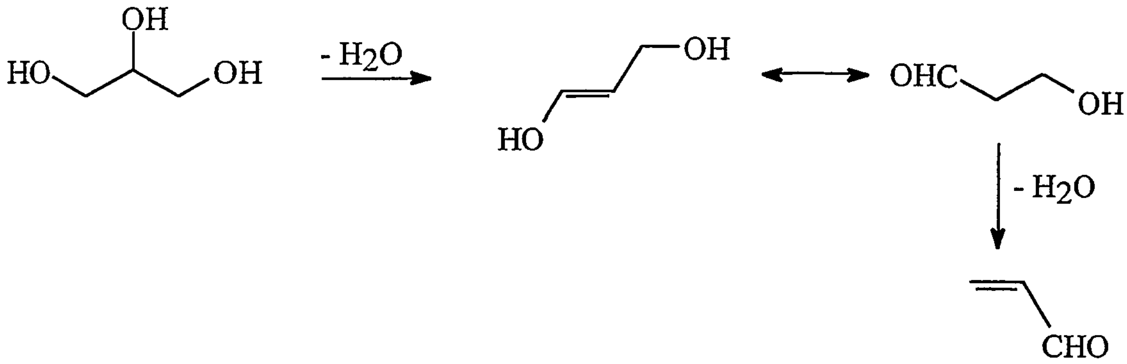


L'acroléine peut brunir le mélange réactionnel et lui conférer une forte odeur tout à fait incompatible avec son utilisation dans l'industrie agro-alimentaire. De plus, l'acroléine est toxique pour le corps humain.

Le processus de polymérisation est généralement mis en oeuvre en présence de catalyseurs basiques, mais il peut parfois être réalisé en milieu acide.

D'après Jungerman (E. JUNGERMANN, *"Glycerine : A key cosmetic ingredient",* Cosmetic Science and Technology Series, Ed. JUNGERMANN, 11 (1991), 97-112), la polymérisation en présence de catalyseurs basiques est généralement effectuée à des températures comprises entre 200 et 270°C. Dans ces conditions, la formation de sous-produits cycliques et branchés est inévitable.

S'agissant de la réaction de polymérisation avec des catalyseurs basiques homogènes, celle-ci peut être effectuée par un mécanisme de type $SN_2$ : l'agent basique a pour rôle d'affaiblir une liaison OH du glycérol et donc d'augmenter la nucléophilie de l'atome d'oxygène selon la réaction suivante :

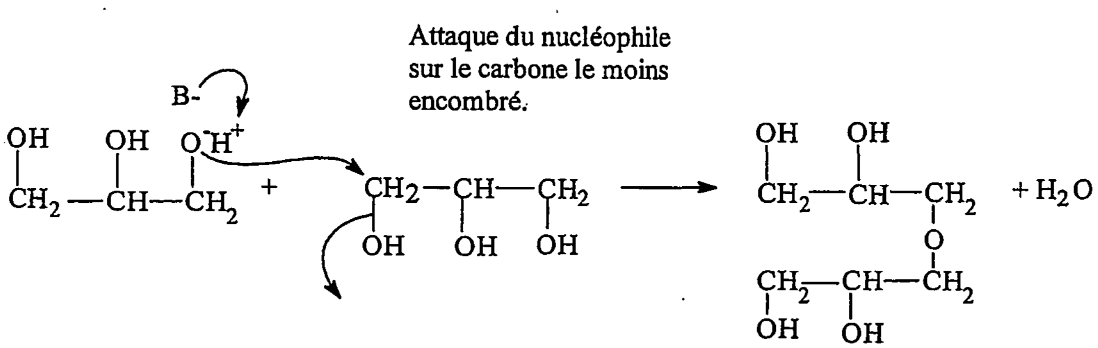


On peut ainsi obtenir un mélange de polyglycérols avec une distribution hétérogène, (demande EP 518,765 au nom d'Onidol, 1992), en utilisant la phase résiduelle (riche en glycérol) issue de la transestérification de triglycérides

en présence de soude ou de potasse à 230-250°C.

L'obtention de polyglycérols à partir du glycérol catalysée par NaOH (250°C) a aussi été décrite dans la demande DE 3,346,097 au nom de Hoechst (1983); l'ajout de $H_3PO_2$ au milieu est recommandé pour éviter le brunissement du polymère final. De même, selon le procédé de Procter et Gamble (brevet US 2,455,327, de 1973), la réaction est effectuée sous pression réduite (130°C/5mm de Hg) afin d'éliminer l'eau formée le plus rapidement possible. En outre, la société Nippon Oils and Fats prépare des di-, tri-, tétraglycérols en effectuant la réaction à 250°C en présence de carbonate de sodium et d'argile (JP 61,238,749).

La synthèse sélective de diglycérol s'effectue à température plus basse (240°C) en présence de silicate de sodium, $SiO_2/Na_2CO_3$ (demandes DE 4,029,323 (1993) et DE 4,117,033 (1991),au nom de Henkel), ou encore avec des composés à base de bore ($NaBO_2$) (brevet US 2,455,327, de 1973, susmentionné).

S'agissant de la réaction de polymérisation du glycérol avec des catalyseurs acides homogènes, il convient de signaler que dans le cas d'une catalyse acide, un mécanisme de type $SN_1$, tel que décrit ci-après, semble plus favorable.

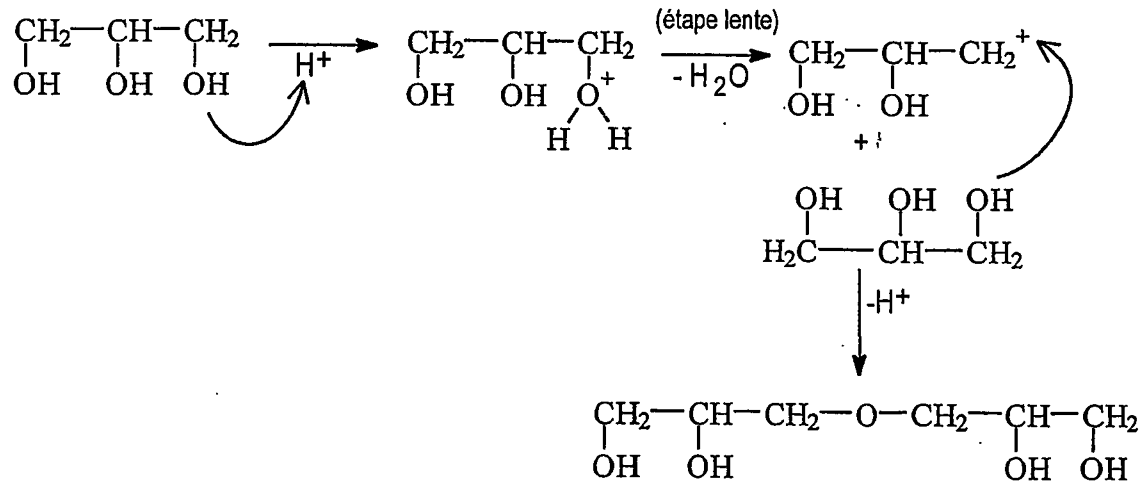


Ainsi, un mélange de polyglycérols a été obtenu (33 % de di, 11 % de tri, 4 % de tétra, 50% de glycérol) en réalisant la réaction sous vide (5mm Hg) à 150°C en présence d'acide dodécylbenzènesulfonique (demande PL 137,052 (1987), au nom de Instytut Chemii Przemtslowej).

De même, Procter et Gamble ont fait réagir le glycérol et l'acétate de glycérol avec $H_2SO_4$ à 130°C sous 5mm de Hg (brevet US 3,968,169, de 1976).

Du point de vue chimique, les catalyseurs homogènes en général, peuvent conduire à des réactions secondaires de déshydratation, d'oxydation, voire à une dégradation des produits (coloration).

De plus l'utilisation de ces catalyseurs homogènes entraîne des étapes de séparation des autres constituants du milieu réactionnel, parfois lourdes à mettre en oeuvre. Par ailleurs, ces catalyseurs homogènes ne sont pas réutilisables car ils nécessitent de coûteuses étapes de lavage, et sont à ce titre nuisibles pour l'environnement.

Afin de pallier ces inconvénients liés à l'utilisation de ces catalyseurs homogènes, des procédés de polymérisation du glycérol à l'aide de catalyseurs solides hétérogènes acides choisis parmi les zéolithes, ont été décrits (brevets US 5,635,588 de Unichema, et US 5,349,094 de Henkel). Il a ainsi pu être observé que les zéolithes, notamment ceux à larges pores (zéolithes β), permettent d'effectuer plus facilement la réaction désirée. Cependant, les réactions secondaires liées à la présence de sites acides conduisent à la formation d'acroléine.

Le tableau I ci-après regroupe les résultats obtenus par les Inventeurs dans le cadre de la polymérisation du glycérol en présence de catalyseurs solides choisis parmi les zéolithes β et A, ou d'un catalyseur homogène, à savoir le carbonate de sodium.

D'après les rendements (à haute conversion), si le carbonate de sodium est le meilleur catalyseur, il n'est cependant pas réutilisable car il nécessite de coûteuses étapes de lavage, et présente l'ensemble des inconvénients susmentionnés.

Tableau 1 : Comparaison des conversions et sélectivités (et rendements) des différents procédés (% massique) conduisant spécifiquement au di- et triglycérol.

| Brevets | Catalyseurs | T (°C) | Conversion du glycérol | Sélectivité en diglycérol (rendement) | Sélectivité en triglycérol (rendement) | Sélectivité en di + tri (rendement) |
|---|---|---|---|---|---|---|
| Unichema | Zéolithe β (20% massique) | 200 | 100[a] | 60(36) [a] | 30(18) [a] | 90(54) [a] |
| Henkel | Zéolithe A (2,4% massique) | 240 | 84,6 | 38,2(32,3) | 24,2 (20,5) | 64,4 (52,8) |
| Homogène | $Na_2CO_3$ (2 % massique) | 260 | 87,5 | 50,4 (44,1) | 26,0 (22,8) | 76,4 (66,9) |
| [a] bilan sur 60% en poids des produits récupérés, donc il reste 40% de produits qui ne sont pas des polyglycérols. | | | | | | |

Les Inventeurs ont testé l'effet de catalyseurs mésoporeux, dans lesquels un élément basique du type Mg ou Al a le cas échéant été incorporé, dans le cadre de l'éthérification du glycérol, en comparaison avec les zéolithes (J. M. Clacens et al., Studies in surface science and catalysis., vol.118, 1998, p895-899). Toutefois, les Inventeurs ont mis en évidence que ces catalyseurs mésoporeux basiques incorporés n'étaient pas suffisamment stables pour permettre une réaction de catalyse hétérogène dans le cadre de l'éthérification du glycérol, dans la mesure où les éléments basiques incorporés dans ces catalyseurs étaient en tout ou partie relargués dans le milieu réactionnel. On se retrouvait donc après un certain temps de fonctionnement dans des conditions de catalyse homogène, et les catalyseurs mésoporeux récupérés après la réaction de catalyse n'étaient en aucun cas recyclables dans la mesure où ils avaient perdu leurs éléments basiques nécessaires au bon déroulement de la réaction de catalyse.

Un des buts de la présente invention est de fournir de nouveaux procédés d'éthérification du glycérol par polymérisation de ce dernier offrant tous les avantages de la catalyse hétérogène, notamment la facilité de récupération du catalyseur, son éventuel recyclage et la possibilité de mettre en oeuvre des procédés continus, tout en présentant, le cas échéant, de meilleurs rendements que ceux des procédés utilisant la catalyse hétérogène décrits jusqu'à maintenant, et évitant la formation de produits de dégradation tel que l'acroléine.

La présente invention a également pour but de fournir de nouveaux procédés susmentionnés dont la sélectivité de formation de di et/ou triglycérol est nettement supérieure à celle des procédés utilisant des catalyseurs homogènes et/ou hétérogènes décrits jusqu'à maintenant.

La présente invention a également pour but de fournir de nouveaux catalyseurs pour la mise en oeuvre des procédés susmentionnés.

L'invention a pour objet l'utilisation de catalyseurs solides de type mésoporeux basiques pour la mise en oeuvre d'un procédé d'éthérification par polymérisation du glycérol ou du glycidol.

L'invention a pour objet l'utilisation de catalyseurs solides de type mésoporeux basiques modifiés par imprégnation, échange, ou greffage d'éléments basiques, pour la mise en oeuvre d'un procédé d'éthérification par polymérisation du glycérol ou du glycidol.

L'invention a plus particulièrement pour objet l'utilisation susmentionnée de catalyseurs solides de type mésoporeux basiques définis ci-dessus, et pour lesquels les éléments basiques imprégnés, échangés, ou greffés, sont choisis parmi les éléments alcalins, et/ou alcalino-terreux, et/ou les terres rares.

Avantageusement, les catalyseurs mésoporeux basiques modifiés susmentionnés ne sont pas solubles dans milieu réactionnel, à savoir dans le glycérol ou le glycidol, dans la mesure où ils ne relarguent pas les éléments basiques imprégnés, échangés ou greffés, dans le milieu réactionnel, ou ils relarguent de tels éléments dans des proportions négligeables, notamment inférieures à environ 10 à 20% en poids de ces éléments basiques, de telle sorte que les catalyseurs demeurent hétérogènes, sont récupérables par simple filtration et sont recyclables.

Par l'expression "catalyseur", on entend tout matériau capable, dans le cadre de la présente invention, d'activer le réactif glycérol et de permettre la réaction susmentionnée sans modification notable de ses propriétés.

Par l'expression "mésoporeux", on entend tout matériau dont la taille des pores est comprise entre environ 15 Å et environ 100 Å.

Par l'expression "solide", on entend tout matériau non ou très peu soluble dans le milieu réactionnel.

Par l'expression "basique", on entend un catalyseur solide présentant préférentiellement à sa surface des centres actifs ayant une tendance à donner un électron ou à accepter une paire d'électrons.

L'invention a plus particulièrement pour objet l'utilisation de catalyseurs solides de type mésoporeux basiques modifiés susmentionnés, pour la mise en oeuvre d'un procédé d'éthérification par polymérisation du glycérol ou du glycidol en vue de l'obtention de compositions comprenant majoritairement du diglycérol et/ou du triglycérol.

A titre d'illustration, les compositions susmentionnées comprenant majoritairement du diglycérol et/ou du triglycérol, sont telles que la proportion en poids de diglycérol et/ou de triglycérol est comprise entre environ 50 % et environ 100%, notamment est d'environ 60 % à environ 80 %, par rapport au poids total des différents constituants, notamment du glycérol et des polyglycérols, desdites compositions. Avantageusement, les compositions susmentionnées sont telles qu'elles comprennent au moins environ 50% de diglycérol et/ou au moins environ 10% de triglycérol, par rapport au poids total desdites compositions.

L'invention a plus particulièrement pour objet l'utilisation susmentionnée de catalyseurs mésoporeux modifiés définis ci-dessus, dont la taille des pores est comprise entre environ 15 Å et environ 50 Å.

L'invention concerne plus particulièrement encore l'utilisation susmentionnée de catalyseurs mésoporeux modifiés définis ci-dessus, dont les éléments constitutifs sont choisis parmi le silicium, le titane, le zirconium, l'aluminium, le bore, le gallium ou le lanthane, ou les mélanges de deux ou plus de ces éléments.

L'invention a plus particulièrement pour objet l'utilisation susmentionnée de catalyseurs mésoporeux modifiés définis ci-dessus, dont les éléments constitutifs sont choisis parmi les matériaux composés de mélange :

- d'une part de silicium, de titane ou de zirconium,
- et, d'autre part, d'aluminium, de bore, de gallium ou de lanthane.

Comme cela a été mentionné ci-dessus, les catalyseurs mésoporeux modifiés susmentionnés, comprennent en plus des éléments constitutifs susmentionnés desdits catalyseurs, des éléments basiques choisis parmi les éléments alcalins, et/ou alcalino-terreux, et/ou les terres rares, ces éléments basiques étant de préférence sélectionnés parmi les suivants :

- les éléments alcalins sont choisis parmi le lithium, le sodium, le potassium, ou le césium,
- les éléments alcalino-terreux sont choisis parmi le magnésium, le calcium, ou le baryum,
- les terres rares sont choisies parmi le lanthane, ou le samarium.

Avantageusement la quantité des éléments alcalins, et/ou alcalino-terreux, et/ou de terres rares, dans les catalyseurs mésoporeux modifiés utilisés dans le cadre de la présente invention, peut atteindre jusqu'à environ 50 % en poids par rapport au poids total du catalyseur, et est de préférence comprise entre environ 10 % et environ 20 %.

De préférence, les catalyseurs mésoporeux susmentionnés utilisés dans le cadre de la présente invention, sont tels qu'obtenus par mise en oeuvre d'un procédé comprenant les étapes suivantes :

- addition d'une solution contenant le ou les éléments constitutifs du matériau mésoporeux, à une solution contenant un agent tensioactif dont le nombre d'atomes de carbone n de la chaîne alkyle est compris entre environ 10 et environ 20, et est de préférence de 16, notamment le bromure d'hexadécyltriméthylammonium (n=16), et, le cas échéant, à une solution contenant un sel d'un ou plusieurs éléments à incorporer,
- chauffage du gel obtenu à l'étape précédente à environ 100°C pendant environ 24 heures,
- calcination du solide obtenu à l'étape précédente à une température finale d'environ 200°C à environ 800°C, de préférence d'environ 400°C à environ 600°C, notamment d'environ 550°C, avantageusement pendant environ 12 heures, ladite température finale étant atteinte par paliers successifs d'environ 0,1°C. $min^{-1}$ à environ 20°C. $min^{-1}$, en partant de la température ambiante, ladite calcination étant avantageusement effectuée sous atmosphère comprenant quelques ppm jusqu'à 100% d'oxygène, de préférence sous air dilué comprenant environ 1% à environ 20% d'oxygène, et éventuellement d'autres gaz tels que l'azote, l'hélium ou l'argon, ce qui conduit à l'obtention d'un catalyseur mésoporeux de formule $M_n$, dans laquelle M représente le matériau mésoporeux support, et n représente le nombre d'atomes de carbone de la chaîne alkyle du tensioactif susmentionné, ou d'un matériau mésoporeux de formule $M_n\ E_r\ (E^x{}_{rx})_y$ dans laquelle y = 0 ou 1, E et $E^x$ représentent les symboles chimiques d'un élément incorporé, r et $r_x$ représentent le rapport molaire entre le matériau mésoporeux et l'élément incorporé (à partir des quantités introduites lors du mélange de ces réactifs), et M et n sont tels que définis ci-dessus,
- modification du catalyseur mésoporeux obtenu à l'étape précédente, de formule $M_n$, ou de formule $M_n\ E_r\ (E^x{}_{rx})_{y,}$, de manière à ce qu'il comprenne des éléments choisis parmi les éléments alcalins, et/ou alcalino-terreux, et/ou les terres rares, tels que définis ci-dessus, par imprégnation, échange, ou greffage de tels éléments.

L'invention a plus particulièrement pour objet l'utilisation susmentionnée, de catalyseurs mésoporeux modifiés par imprégnation avec un ou plusieurs éléments ne réagissant pas chimiquement avec le ou les matériaux constitutifs desdits catalyseurs.

De préférence, la modification des catalyseurs mésoporeux par imprégnation comprend les étapes suivantes :

- mélange en solution du catalyseur mésoporeux obtenu après l'étape de calcination selon le procédé décrit ci-dessus,

avec un sel du ou des éléments à imprégner,
- calcination du solide obtenu après évaporation du solvant utilisé dans l'étape de mélange précédente, dans les conditions de calcination indiquées ci-dessus.

A ce titre, l'invention a pour objet l'utilisation de catalyseurs mésoporeux imprégnés susmentionnés, répondant à la formule générale

$$E_I\ (E^x{}_{Ix})_y\ M_n$$

dans laquelle :

- y = 0 ou 1,
- E et $E^x$ représentent les symboles chimiques d'un élément imprégné,
- I et $I_x$ représentent le nombre de moles d'élément mises en jeu lors de l'imprégnation (x$10^{-4}$ et par gramme de support), et sont avantageusement compris entre environ 0,1 et environ 100 selon l'élément considéré,
- M représente le matériau mésoporeux support,
- n représente le nombre d'atomes de carbone de la chaîne alkyle du tensioactif utilisé dans la préparation du matériau mésoporeux, notamment n= 16 dans le cas de l'utilisation de bromure d'hexadécyltriméthylammonium.

Des catalyseurs imprégnés préférés répondant à la formule générale susmentionnée sont choisis parmi les suivants :
$Li_{25}\ M_{16}$, $La_{25}\ M_{16}$, $Cs_{25}\ M_{16}$, $Na_{25}\ M_{16}$, $Mg_{25}\ M_{16}$, $Cs_{100}\ M_{16}$, $Cs_{50}\ M_{16}$, $Cs_6\ M_{16}$, $Cs_6\ La_6\ M_{16}$, $Cs_{12}\ La_{12}\ M_{16}$, dans lesquels le matériau mésoporeux M est de préférence choisi parmi ceux contenant du silicium et/ou de l'aluminium.
L'invention a plus particulièrement pour objet l'utilisation susmentionnée des catalyseurs mésoporeux imprégnés définis ci-dessus, et répondant à la formule générale suivante :

$$E_I\ (E^x{}_{Ix})_y\ M_n\ Al_r$$

dans laquelle :

- y = 0 ou 1,
- E et $E^x$ représentent les symboles chimiques d'un élément imprégné,
- I et $I_x$ représentent le nombre de moles d'élément mises en jeu lors de l'imprégnation (x$10^{-4}$ et par gramme de support), et sont avantageusement compris entre environ 0,1 et environ 100 selon l'élément considéré,
- M représente le matériau mésoporeux support,
- n représente le nombre d'atomes de carbone de la chaîne alkyle du tensioactif utilisé dans la préparation du matériau mésoporeux, notamment n= 16 dans le cas de l'utilisation de bromure d'hexadécyltriméthylammonium,
- r représente le rapport molaire Si/Al, à partir des quantités d'aluminium introduites lors du mélange des réactifs lors de l'étape d'imprégnation dans le procédé décrit ci-dessus, dans le cas où M représente un aluminosilicate mésoporeux, et est avantageusement compris entre environ 0 et environ 100.

Des catalyseurs imprégnés préférés répondant à la formule générale susmentionnée sont choisis parmi les suivants :
$Li_{25}\ M_{16}\ Al_{20}$, $La_{25}\ M_{16}\ Al_{20}$, $Cs_{25}\ M_{16}\ Al_{20}$, $Na_{25}\ M_{16}\ Al_{20}$, $Mg_{25}\ M_{16}\ Al_{20}$, $Cs_{100}\ M_{16}\ Al_{20}$, $Cs_{50}\ M_{16}\ Al_{20}$, $Cs_6\ M_{16}\ Al_{20}$, $Cs_6\ La_6\ M_{16}\ Al_{20}$, $Cs_{12}\ La_{12}\ M_{16}\ Al_{20}$, dans lesquels le matériau mésoporeux M contient du silicium et de l'aluminium.
L'invention a pour objet l'utilisation susmentionnée des catalyseurs mésoporeux imprégnés plus particulièrement préférés suivants :
$Cs_{25}\ M_{16}$, $Mg_{25}\ M_{16}\ Al_{20}$, $Cs_{25}\ M_{16}\ Al_{20}$, $La_{25}\ M_{16}\ Al_{20}$, $Cs_{12}\ La_{12}\ M_{16}\ Al_{20}$, $Na_{25}\ M_{16}\ Al_{20}$, dans lesquels le matériau mésoporeux M contient du silicium et/ou de l'aluminium.
L'invention a également pour objet l'utilisation susmentionnée, de catalyseurs mésoporeux modifiés par greffage avec un ou plusieurs éléments capables de se lier chimiquement avec le ou les matériaux constitutifs desdits catalyseurs, notamment par le biais de liaisons du type partiellement ionique.
De préférence, la modification des catalyseurs mésoporeux par greffage comprend les étapes suivantes :

- mélange d'un sel du ou des éléments à greffer avec le matériau mésoporeux support utilisé tel qu'obtenu après calcination selon le procédé décrit ci-dessus, notamment de manière à obtenir un rapport M (matériau support) / élément à greffer d'environ 20, avantageusement dans un solvant choisi parmi l'éthanol absolu, ou l'hexane,
- calcination du solide obtenu après évaporation du solvant utilisé dans l'étape de mélange précédente, dans les

conditions de calcination indiquées ci-dessus.

L'invention a plus particulièrement pour objet l'utilisation susmentionnée des catalyseurs mésoporeux greffés définis ci-dessus, et répondant à la formule générale suivante :

$$E_{Ig}\,M_n$$

dans laquelle :

- E représente le symbole chimique d'un élément greffé,
- I représente le nombre de moles d'élément mises en jeu lors du greffage ($x10^{-4}$ et par gramme de support), et est avantageusement compris entre environ 5 et environ 20,
- g signifie que l'élément est greffé,
- M représente le matériau mésoporeux support,
- n représente le nombre d'atomes de carbone de la chaîne alkyle du tensioactif utilisé dans la préparation du matériau mésoporeux, notamment n= 16 dans le cas de l'utilisation de bromure d'hexadécyltriméthylammonium.

L'invention a pour objet l'utilisation susmentionnée des catalyseurs mésoporeux greffés plus particulièrement préférés suivants :

$Cs_{Ig}[Si\text{-}M_{16}]$, $Al_{Ig}[Si\text{-}M_{16}]$, dans lesquels $Si\text{-}M_{16}$ signifie que le matériau mésoporeux contient que du silicium.

L'invention a également pour objet l'utilisation susmentionnée, de catalyseurs mésoporeux modifiés par échange avec un ou plusieurs éléments capables de remplacer des éléments constituant le ou les matériaux constitutifs desdits catalyseurs.

De préférence, la modification des catalyseurs mésoporeux par échange comprend les étapes suivantes :

- mélange d'un sel du ou des éléments à échanger avec le matériau mésoporeux support utilisé tel qu'obtenu après calcination selon le procédé décrit ci-dessus, notamment de manière à obtenir un rapport M (matériau support) / élément à échanger d'environ 20, avantageusement dans un solvant choisi parmi l'éthanol absolu, ou l'hexane,
- calcination du solide obtenu après évaporation du solvant utilisé dans l'étape de mélange précédente, dans les conditions de calcination indiquées ci-dessus.

L'invention a plus particulièrement pour objet l'utilisation susmentionnée des catalyseurs mésoporeux échangés définis ci-dessus, et répondant à la formule générale suivante :

$$E_{Ie}\,M_n$$

dans laquelle :

- E représente le symbole chimique d'un élément échangé,
- I représente le nombre de moles d'élément mises en jeu lors de l'échange ($x10^{-4}$ et par gramme de support), et est avantageusement compris entre environ 5 et environ 20,
- e signifie que l'élément est échangé,
- M représente le matériau mésoporeux support,
- n représente le nombre d'atomes de carbone de la chaîne alkyle du tensioactif utilisé dans la préparation du matériau mésoporeux, notamment n=16 dans le cas de l'utilisation de bromure d'hexadécyltriméthylammonium.

L'invention a pour objet l'utilisation susmentionnée des catalyseurs mésoporeux échangés plus particulièrement préférés suivants :

$Cs_{Ie}[Si\text{-}M_{16}]$, dans lesquels $Si\text{-}M_{16}$ signifie que le matériau mésoporeux ne contient que du silicium.

L'invention a pour objet l'utilisation de catalyseurs mésoporeux basiques susmentionnés modifiés par imprégnation d'un ou plusieurs éléments, et/ou par greffage d'un ou plusieurs éléments, et/ou par échange d'un ou plusieurs éléments, notamment selon les procédés susmentionnés.

A ce titre, l'invention a plus particulièrement pour objet l'utilisation susmentionnée des catalyseurs mésoporeux échangés et greffés plus particulièrement préférés suivants :

$Cs_{Ie}Al_g[Si\text{-}M_{16}]$, dans lesquels $Si\text{-}M_{16}$ signifie que le matériau mésoporeux ne contient que du silicium, I, e, et g étant tels que définis ci-dessus.

L'invention a également pour objet un procédé d'éthérification par polymérisation du glycérol ou du glycidol, caractérisé en ce qu'il comprend la mise en présence de glycérol, de glycérine, ou de glycidol, avec un catalyseur solide

mésoporeux basique modifié tel que défini ci-dessus.

Avantageusement, la quantité de catalyseur utilisée dans le procédé susmentionné, est comprise entre environ 0,001% et environ 50% en poids par rapport au poids de glycérol, glycérine ou glycidol de départ.

De préférence, la température de réaction dudit procédé est comprise entre environ 100°C et 300°C, et de préférence comprise entre environ 150°C et environ 250°C.

De préférence également, ledit procédé est effectué sous pression atmosphérique, ou sous pression réduite, notamment jusqu'à environ $7.10^{-3}$ bar, ou sous pression supérieure à la pression atmosphérique, notamment jusqu'à 50 bar, dans l'air ambiant ou sous atmosphère contrôlée.

L'invention a également pour objet l'application du procédé susmentionné à l'obtention de diglycérol et/ou de triglycérol, ledit procédé comprenant, le cas échéant, une étape supplémentaire de séparation d'une part du diglycérol et/ou du triglycérol, et d'autre part du glycérol restant et éventuellement des autres polyglycérols formés au cours de la réaction.

L'invention concerne également les catalyseurs mésoporeux modifiés suivants :

$M_{16}$, $M_{16}$ $Al_{20}$, $M_{16}$ $Li_{20}$, $M_{16}$ $Mg_{20}$, $M_{16}$ $Cs_{20}$, $M_{16}$ $Mn_{20}$, $M_{16}$ $Co_{20}$, $M_{16}$ $La_{20}$, $M_{12}$ $La_{20}$, $Li_{25}$ $M_{16}$, $La_{25}$ $M_{16}$, $Cs_{25}$ $M_{16}$, $Na_{25}$ $M_{16}$, $Mg_{25}$ $M_{16}$, $Cs_{100}$ $M_{16}$, $Cs_{50}$ $M_{16}$, $Cs_6$ $M_{16}$, $Cs_6$ $La_6$ $M_{16}$, $Cs_{12}$ $La_{12}$ $M_{16}$, $Li_{25}$ $M_{16}$ $Al_{20}$, $La_{25}$ $M_{16}$ $Al_{20}$, $Cs_{25}$ $M_{16}$ $Al_{20}$, $Na_{25}$ $M_{16}$ $Al_{20}$, $Mg_{25}$ $M_{16}$ $Al_{20}$, $Cs_{100}$ $M_{16}$ $Al_{20}$, $Cs_{50}$ $M_{16}$ $Al_{20}$, $Cs_6$ $M_{16}$ $Al_{20}$, $Cs_6$ $La_6$ $M_{16}$ $Al_{20}$, $Cs_{12}$ $La_{12}$ $M_{16}$ $Al_{20}$.

L'invention a plus particulièrement pour objet les catalyseurs mésoporeux modifiés susmentionnés dans lesquels le matériau mésoporeux M est de préférence choisi parmi ceux contenant du silicium et/ou de l'aluminium, et est notamment un aluminosilicate dans lequel la proportion de silice est comprise entre environ 0% et environ 100%, pour environ 100% à environ 0% d'aluminium, par rapport au poids total de l'aluminosilicate.

L'invention sera davantage illustrée à l'aide des exemples qui suivent de préparation de catalyseurs mésoporeux tels que définis ci-dessus selon l'invention, et de l'utilisation de ces derniers dans le cadre de la mise en oeuvre d'un procédé d'éthérification par polymérisation du glycérol selon l'invention.

## I) CATALYSEURS

### *1) Préparation*

*1.1. Matériaux mésoporeux supports obtenus par synthèse directe*

Les catalyseurs mésoporeux (M) supports, le cas échéant incorporés avec différents éléments, ont été préparés selon un mode opératoire élaboré au laboratoire à partir de ceux décrits dans l'article de J. C. Vartuli et J. S. Beck, Nature, 359 (1992) 710.

On prépare une solution contenant 0,022 mole de NaOH, 23,31 moles d'eau, 0,005 mole de nitrate de l'élément à incorporer et 0,043 mole de tensioactif. Cette solution est chauffée à environ 40°C jusqu'à obtention d'un mélange transparent. On y ajoute, goutte à goutte, 0,2 mole d'une solution de silicate de sodium (27% $SiO_2$). Le pH est ajusté à 10,5 avec HCl dilué tout au long de l'ajout. Le gel formé est ensuite transféré dans un autoclave en téflon et placé dans une étuve à 100°C pendant 24 heures. Le solide résultant est filtré, lavé à l'eau distillée, séché à 100°C pendant une nuit. Il est finalement calciné sous air à 550°C (montée en température de $1°C.\ min^{-1}$) pendant 6 heures (palier), ce qui conduit à l'obtention d'un catalyseur mésoporeux de formule $M_n$, ou de formule $M_n$ $E_r$ $(E^x{}_{rx})_y$ telles que définies ci-dessus.

*1.2. Matériaux mésoporeux modifiés par imprégnation*

Pour les catalyseurs mésoporeux imprégnés par différents éléments, la méthode utilisée dérive des méthodes décrites par Kloetstra (K.R. KLOETSTRA et H. VAN BEKKUM, *J. Chem. Soc. Chem. Commun.* (1995), 1005; K.R. KLOETSTRA et H. VAN BEKKUM, *Stud. Surf. Sci. Catal.*, **105** (1997), 431; K.R. KLOETSTRA, M. VAN LAREN et H. VAN BEKKUM, *J. Chem. Soc. Faraday Trans.*, **93** (1997), 1211; K.R. KLOETSTRA et H. VAN BEKKUM, *Catal. Lett.,* **47** (1997), 235).

La préparation consiste à placer sous agitation pendant deux heures une solution contenant : 5 g d'un silicate ou d'un aluminosilicate mésoporeux calciné (servant de support), 50 g de méthanol et 0,01457 mole de sel de l'élément à imprégner (CsAc, $Mg(NO_3)_2.6H_2O$, $La(NO_3)_3.6H_2O$, ...). Ensuite, le solvant est évaporé rapidement sous vide et séché à 100°C (1 heure), puis calciné sous air à 450°C (montée en température de $1°C.min^{-1}$) pendant 6 heures (palier).

Ces échantillons seront répertoriés selon la nomenclature suivante :

$$E_l\ (E^x{}_{lx})_y\ M_n\ Al_r$$

dans laquelle :

- y = 0 ou 1,
- E et $E^x$ représentent les symboles chimiques d'un élément imprégné,
- I et $I_x$ représentent le nombre de moles d'élément mises en jeu lors de l'imprégnation ($x10^{-4}$ et par gramme de support), et sont avantageusement compris entre environ 0,1 et environ 100,
- M = matériau mésoporeux
- n = le nombre d'atomes de carbone de la chaîne alkyle du tensioactif (16 pour le bromure de cétyltriméthylammonium)
- r = le rapport molaire Si / Al (à partir des quantités d' aluminium introduites lors du mélange des réactifs ; dans le cas des aluminosilicates)

*1.3. Matériaux mésoporeux modifiés par greffage*

Les matériaux mésoporeux qui ont servi pour le greffage sont ceux décrits dans l'article de A. KARLSSON, M. STOCKER et R. SCHMIDT, *Microporous and Mesoporous Mater.,* **27** (1999), 181-192. Il s'agit de MCM-41 ne contenant que du silicium (appelé Si-MCM-41) et des systèmes mixtes (MCM-41/ZSM5) contenant du silicium et de l'aluminium (appelé A1-MCM-41/ZSM5).

Nous avons tenté de greffer de l'aluminium sur les solides ne contenant que du silicium. La procédure de greffage de l'aluminium, dérivée du travail de Mokaya (R. MOKAYA et W. JONES, *Chem. Commun.* (1997),2185-2186) est la suivante :

A une solution contenant 0,34 g d'isopropoxyde d'aluminium et 150 ml d'hexane, on ajoute une suspension contenant 2 g de Si-MCM-41 (ou Si-MCM/ZSM5) et 50 ml d'hexane de manière à obtenir un rapport Si/Al de 20. Après 10 minutes d'agitation à température ambiante sous atmosphère inerte (Ar), le mélange est laissé au repos 24 heures à cette température sous argon. On filtre ensuite le mélange ; le solide résultant est lavé avec de l'hexane et puis calciné sous air (1h00 à 100°C et 4h00 à 550°C avec une montée en température de $5°C.min^{-1}$).

Ces matériaux seront répertoriés selon la nomenclature suivante : $Al_g$Si-MCM-41 et $Al_g$Si-MCM41/ZSM5.

Du césium a également été greffé sur les solides ne contenant que du silicium. Pour cela, deux types de synthèse ont été mises en oeuvre ; l'une utilisant l'éthanol absolu comme solvant, dérivée des travaux de Ryoo (R. RYOO, S. JUN, J.M. Kim et M.J. KIM, *Chem. Commun.* (1997), 2225-2226), et l'autre utilisant l'hexane comme solvant.

- Dans le premier type de synthèse, une suspension contenant 2 g de Si-MCM-41 ou (Si-MCM-41/ZSM5) et 50 ml d'éthanol absolu est ajoutée à une solution contenant 0,32 g de nitrate de césium et 150 ml d'éthanol absolu de manière à obtenir un rapport Si/Cs de 20. Après une heure d'agitation à température ambiante, le solide est filtré, lavé à l'éthanol absolu et calciné sous air (1h00 à 100°C et 4h00 à 550°C avec une montée en température de $5°C.min^{-1}$). Ces matériaux seront dénommés selon la nomenclature suivante : $Cs_g$Si-MCM-41(I) et $Cs_g$Si-MCM-41/ZSM5(I).
- Dans le deuxième type de synthèse, à une solution contenant 0,32 g de nitrate de césium et 150 ml d'hexane, on ajoute une suspension contenant 2g de Si-MCM-41 (ou Si-MCM-41/ZSM5) et 50 ml d'hexane de manière à obtenir un rapport Si/Cs de 20. Après 10 minutes d'agitation à température ambiante sous atmosphère inerte (Ar), le mélange est laissé au repos 24 heures à cette température et sous argon. On filtre ensuite le mélange ; le solide résultant est lavé avec de l'hexane et puis calciné sous air (1h00 à 100°C et 4h00 à 550°C avec une montée en température de $5°C.min^{-1}$).

Ces matériaux seront répertoriés selon la nomenclature suivante : $Cs_g$Si-MCM-41(II) et $Cs_g$Si-MCM-41/ZSM5 (II).

Afin de comparer les différents modes d'incorporation du césium, nous avons également élaboré des échantillons par échange et par imprégnation de césium. Ces synthèses ont été effectuées avec les supports Al-MCM-41/ZSM5, $Al_g$Si-MCM-41 et $Al_g$Si-MCM-41/ZSM5.

- En ce qui concerne l'échange de césium, 2g de solide mésoporeux ont été mélangés pendant 20h00 à température ambiante dans 20 ml d'une solution éthanolique anhydre contenant 0,39 g de nitrate de césium (solution 0,1M). Après filtration et lavage à l'éthanol absolu, les solides ont été calcinés sous air à 500°C pendant 5h00 (montée en température = $5°C.min^{-1}$).

Ces matériaux seront répertoriés selon la nomenclature suivante : $Cs_e$Al-MCM-41/ZSM5, $Cs_eAl_g$Si-MCM-41 et $Cs_eAl_g$Si-MCM-41/ZSM5.

- Pour l'imprégnation du césium, 2 g de solide mésoporeux ont été mélangés pendant 20h00 à température ambiante dans une solution contenant 3,84 g d'acétate de césium (sec) et 20 ml d'éthanol absolu (solution 1M). Après filtration, les solides ont été directement calcinés sous air à 500°C pendant 5h00 (montée en température = $5°C.min^{-1}$).

Ces matériaux seront répertoriés selon la nomenclature suivante : $Cs_i$Al-MCM-41/ZSM5.

***2) Caractérisation.***

Afin de caractériser les catalyseurs, nous avons effectué différentes analyses physico-chimiques : mesure de surfaces spécifiques (BET et isotherme d'adsorption d'azote), diffraction des rayons X (DRX), dosage d'éléments, microscopie électronique à transmission (MET) couplée à des mesures d'EDX, analyse thermogravimétrique (ATD-ATG) et analyse de surface (XPS).

Le principe de ces techniques est décrit brièvement ci-dessous.

*2.1 Mesures des surfaces spécifiques*

Les mesures des surfaces spécifiques des catalyseurs calcinés ont été réalisées à l'aide d'un appareil Micromeritics Flowsorb 2300 par application de la méthode BET.

Des isothermes d'adsorption/désorption d'azote ont également été effectués avec un appareil Micromeritics ASAP 2010.

*2. 2 Diffraction des rayons X*

La diffraction des rayons X permet d'identifier la nature, la structure et la pureté des matériaux synthétisés.

Le principe est basé sur la loi de Braag :

$2d\sin^{\theta} = n^{\lambda}$, avec $^{\lambda}$ = longueur des rayons X incidents

n = ordre de diffraction

d = interdistance entre les plans réticulaires

$\theta$ = angle de réflexion

La diffraction a été principalement utilisée pour déterminer le diamètre des pores des MCM-41, ainsi que leur type d'agencement (hexagonal, cubique, lamellaire ou amorphe).

L'appareillage utilisé est un Bruker D5005. La source de rayons X est une anticathode de cuivre bombardée par un faisceau généré par une différence de potentiel de 40 kV, d'une intensité de 30 mA. Les raies $K_{\alpha 1}$ et $K_{\alpha 2}$ du Cu sont sélectionnées grâce à un monochromateur au graphite ($\lambda$ $K_{\alpha}$ = 1.5056 Å). Les rayons X diffractés sont détectés par un détecteur à scintillations. Pour les mesures aux bas angles (entre 1° et 6°), on utilise une fente collimatrice de 0,3° entre l'échantillon et la source, ainsi que entre l'échantillon et le détecteur afin de diminuer l'intensité des rayons incidents rasants non réfléchis.

*2. 3 Dosage d'éléments*

Les échantillons sont envoyés au centre d'analyse du CNRS afin de connaître leur composition chimique. Ces analyses sont effectuées après modification des catalyseurs afin de vérifier leur taux d'incorporation, d'échange ou d'imprégnation ; et après réaction, afin de connaître la quantité d'élément restant dans le catalyseur.

*2.4 Microscopie électronique à transmission*

Cette technique permet d'accéder à des informations sur la taille des particules de solide mésoporeux ainsi que sur l'organisation des canaux et sur la présence éventuelle de cristallites d'oxyde ou de métal. En outre, l'analyse EDX (Energy Dispersive spectroscopy ray-X) effectuée sur ces matériaux, permettra d'apprécier la présence et l'éventuelle dispersion de ces cristallites, ainsi que leur nature.

Les analyses sont effectuées à l'aide d'un appareil Philips CM 120 muni d'un filament en $LaB_6$. Les solides mésoporeux sont d'abord inclus dans une résine, des coupes sont alors réalisées grâce à un microtome. Ces coupes sont alors déposées sur une grille afin d'être analysées. Suivant l'orientation aléatoire des particules dans la résine, la coupe peut faire apparaître les canaux dans le sens de la longueur ou bien perpendiculairement à celle-ci.

*2.5. Analyses thermogravimétriques*

Les analyses thermogravimétriques ont été principalement utilisées dans ce travail afin de quantifier les espèces organiques (polyglycérols) restant à l'intérieur du catalyseur récupéré après test catalytique.

Ces analyses ont été réalisées sur un appareil SDT 2960 de TA Instruments qui permet les mesures simultanées de l'analyse thermodifférentielle (ATD) et de l'analyse thermogravimétrique (ATG). Les échantillons sont chauffés sous flux d'air (100 $cm^3.min^{-1}$) à une vitesse de $5°C.min^{-1}$, et ce jusqu'à une température de 550°C avec un palier d'une heure.

*2. 6 Analyses XPS*

Le principe de cette technique d'analyse chimique de la surface des solides repose sur l'effet photoélectrique. En effet, l'irradiation du solide par des rayons X de faible énergie provoque l'émission d'électrons caractérisés par leur énergie cinétique (Ec). Celle-ci dépend de l'énergie de liaison (El) des électrons, propre à chaque élément étudié et de l'énergie des photons incidents (hv).

$$Ec = hv - El$$

Donc en utilisant un rayonnement incident monochromatique, l'énergie cinétique observée dépendra exclusivement de l'énergie de liaison des électrons. Les électrons émis par différents niveaux électroniques de l'atome donnent alors naissance à différents pics. La position de ces pics ainsi que leur surface permettent l'identification des espèces présentes dans leur quantification.

Les analyses XPS ont été effectuées à l'aide d'un appareil SSI (Surface Science Instruments) modèle 301.

II) TEST CATALYTIQUE

***1) Montage***

La réaction est effectuée dans un ballon de 250 ml à cinq cols équipé :

* d'un système d'agitation mécanique (agitateur bipale en Pyrex)
* d'un Dean-Stark (extracteur d'eau) associé à un réfrigérant droit
* d'une arrivée d'azote
* d'un tube plongeur servant de réceptacle à une sonde de température
* d'un système de prélèvement d'échantillons

Le ballon (calorifugé) par de la laine de verre est porté à la température désirée.

***2) Mode opératoire***

Sauf mentions particulières, les conditions opératoires usuelles sont les suivantes :

| | |
|---|---|
| Température : | 260°C |
| Quantité de glycérol : | 50g (0,54mol) |
| Masse de catalyseur : | 1 g |
| Concentration en catalyseur : | 2 % en poids (par rapport au glycérol) |
| Vitesse d'agitation : | 500 tours par minute |

Le glycérol est d'abord porté à la température de réaction sous atmosphère inerte ($N_2$) avant d'ajouter le catalyseur. Ceci correspond au temps zéro de la réaction. Le montage est laissé sous atmosphère d'azote durant toute la réaction afin d'éviter l'oxydation du glycérol. Le suivi du mélange réactionnel est effectué tout au long de la réaction grâce au système de prélèvement. Les échantillons sont analysés par chromatographie en phase gazeuse après silylation.

***3) Récupération des catalyseurs***

Les catalyseurs utilisés pour effectuer la réaction d'éthérification du glycérol sont récupérés par filtration, après dilution du résidu de manipulation dans l'éthanol à 95 % (Carlo Erba). Le solide récupéré est ensuite séché à l'étuve à 100°C. L'alcool contenu dans le filtrat est évaporé au rotavapor.

***4) Caractéristiques du glycérol***

Le glycérol utilisé est un produit Prolabo (pureté : 98 %)

***5) Analyse chromatographique en phase gazeuse***

L'analyse du mélange réactionnel au cours du temps permet de préciser la nature des polyglycérols formés, ainsi que leur quantité relative. La chromatographie en phase gazeuse (CPG) nous a servi à quantifier les produits de réaction.

***6) Résultats***

Les catalyseurs utilisés sont des solides mésoporeux modifiés par échange, imprégnation ou greffage avec des éléments alcalins, alcalino-terreux ou terres rares, obtenus par mise en oeuvre des procédés décrits ci-dessus.
Conditions de réaction : la réaction d'éthérification du glycérol est effectuée sans solvant à une température comprise entre 150 et 300°C ; les meilleurs résultats ayant été obtenus entre 220 et 270°C. Le pourcentage massique de catalyseur utilisé lors de la réaction peut varier de 0.01% à 20 % avec de meilleurs résultats pour un pourcentage variant de 0.5 à 10%.

Exemple 1 :

La réaction est effectuée à pression atmosphérique dans un réacteur de 250 ml équipé d'un système d'élimination d'eau (dean-stark) et à une température de 260°C, sous agitation mécanique et sous atmosphère d'azote. La masse de glycérol est de 50g et la masse de catalyseur est de 1g.
Le catalyseur est un solide mésoporeux imprégné avec de l'acétate de césium ($19.10^{-4}$ mole de césium par gramme de catalyseur), à savoir le catalyseur mésoporeux de formule suivante : $Cs_{25}M_{16}Al_{20}$. Le diamètre des pores du catalyseur est d'environ 4nm. Après 24 heures de réaction, la conversion du glycérol est de 81 % massique.
Les sélectivités et rendements en polyglycérols (à 80 % de conversion massique du glycérol) sont reportés dans le tableau suivant.

| Polyglycérols | Glycérol | Diglycérol | Triglycérol | Tétraglycérol | Autres |
|---|---|---|---|---|---|
| Sélectivité (% massique) | - | 76 | 22 | 2 | 0 |
| Rendement (% massique) | 20 | 60,8 | 17,6 | 1,6 | 0 |

Exemple 2 :

Le protocole expérimental est identique à celui de l'exemple 1.
Le catalyseur est un solide mésoporeux greffé avec du césium ($8.10^{-4}$ mole de césium par gramme de catalyseur), à savoir le catalyseur mésoporeux de formule suivante : $Cs_g$-Si-MCM41. Le diamètre des pores du catalyseur est d'environ 3,2 nm.
Après 48 heures de réaction, la conversion du glycérol est de 56 % massique.
Les sélectivités et rendements en polyglycérols (à 56 % de conversion massique du glycérol) sont reportés dans le tableau suivant.

| Polyglycérols | Glycérol | Diglycérol | Triglycérol | Tétraglycérol | Autres |
|---|---|---|---|---|---|
| Sélectivité (% massique) | - | 86 | 14 | 0 | 0 |
| Rendement (% massique) | 44 | 48,2 | 7,8 | 0 | 0 |

Exemple 3:

Le protocole expérimental est identique à celui de l'exemple 1.
Le catalyseur est un solide mésoporeux échangé avec du césium ($9.10^{-4}$ mole de césium par gramme de catalyseur), à savoir le catalyseur mésoporeux de formule suivante : $Cs_eAl_g$-Si-MCM41/ZSM5. Le diamètre des pores du catalyseur est d'environ 4nm. Après 32 heures de réaction, la conversion du glycérol est de 81 % massique.
Les sélectivités et rendements en polyglycérols (à 80% de conversion massique du glycérol) sont reportés dans le tableau suivant.

| Polyglycérols | Glycérol | Diglycérol | Triglycérol | Tétraglycérol | Autres |
|---|---|---|---|---|---|
| Sélectivité (% massique) | - | 66 | 26 | 7 | 1 |
| Rendement (% massique) | 20 | 52,8 | 20,8 | 5,6 | 0,8 |

**Revendications**

1. Utilisation de catalyseurs solides de type mésoporeux basiques modifiés par imprégnation, échange, ou greffage d'éléments basiques, pour la mise en oeuvre d'un procédé d'éthérification par polymérisation du glycérol ou du glycidol, lesdits éléments basiques imprégnés, échangés ou greffés étant choisis parmi les éléments alcalins, et/ou alcalino-terreux, et/ou les terres rares.

2. Utilisation selon la revendication 1, **caractérisée en ce que** les catalyseurs mésoporeux basiques modifiés ne sont pas solubles dans le glycérol ou le glycidol, à savoir qu'ils ne relarguent pas les éléments basiques imprégnés, échangés ou greffés, dans le milieu réactionnel, de telle sorte que les catalyseurs demeurent hétérogènes, sont récupérables par simple filtration et sont recyclables.

3. Utilisation selon l'une des revendications 1 ou 2, **caractérisée en ce que** les catalyseurs mésoporeux ont des pores dont la taille est comprise entre environ 15 Å et environ 100 Å, et de préférence entre environ 15 Å et environ 50 Å.

4. Utilisation selon l'une des revendications 1 à 3, de catalyseurs mésoporeux modifiés comprenant en plus des éléments constitutifs desdits catalyseurs, des éléments choisis parmi :

   - les éléments alcalins, tels que le lithium, le sodium, le potassium, ou le césium,
   - et/ou les alcalino-terreux, tels que le magnésium, le calcium, ou le baryum,
   - et/ou les terres rares, tels que le lanthane, ou le samarium,

   la quantité des éléments alcalins, et/ou alcalino-terreux, et/ou de terres rares, pouvant atteindre jusqu'à environ 50 % en poids par rapport au poids total du catalyseur, et est notamment comprise entre environ 10 % et environ 20 %.

5. Utilisation selon l'une des revendications 1 à 4, de catalyseurs mésoporeux tels qu'obtenus par mise en oeuvre d'un procédé comprenant les étapes suivantes :

   - addition d'une solution contenant le ou les éléments constitutifs du matériau mésoporeux, à une solution contenant un agent tensioactif dont le nombre d'atomes de carbone de la chaîne alkyle est compris entre environ 10 et environ 20, et est de préférence de 16, notamment le bromure d'hexadécyltriméthylammonium, et, le cas échéant à une solution contenant un sel d'un ou plusieurs éléments à incorporer,
   - chauffage du gel obtenu à l'étape précédente à environ 100°C pendant environ 24 heures,
   - calcination du solide obtenu à l'étape précédente à une température finale d'environ 200°C à environ 800°C, de préférence d'environ 400°C à environ 600°C, notamment d'environ 550°C, avantageusement pendant environ 12 heures, ladite température finale étant atteinte par paliers successifs d'environ 0,1°C.$min^{-1}$ à environ 20°C.$min^{-1}$, en partant de la température ambiante, ladite calcination étant avantageusement effectuée sous atmosphère comprenant quelques ppm jusqu'à 100% d'oxygène, de préférence sous air dilué comprenant environ 1% à environ 20% d'oxygène, et éventuellement d'autres gaz tels que l'azote, l'hélium ou l'argon, ce qui conduit à l'obtention d'un catalyseur mésoporeux de formule $M_n$, dans laquelle M représente le matériau mésoporeux support, et n représente le nombre d'atomes de carbone de la chaîne alkyle du tensioactif susmentionné, ou d'un matériau mésoporeux de formule $M_n\ E_r\ (E^x{}_{rx})_y$ dans laquelle y = 0 ou 1, E et $E^x$ représentent les symboles chimiques d'un élément incorporé, r et $r_x$ représentent le rapport molaire entre le matériau mésoporeux et l'élément incorporé (à partir des quantités introduites lors du mélange de ces réactifs), et M et n sont tels que définis ci-dessus,
   - modification du catalyseur mésoporeux obtenu à l'étape précédente, de formule $M_n$, ou de formule $M_n\ E_r\ (E^x{}_{rx})_y$, de manière à ce qu'il comprenne des éléments choisis parmi les éléments alcalins, et/ou alcalino-terreux, et/ou les terres rares.

6. Utilisation selon l'une des revendications 1 à 5, de catalyseurs mésoporeux imprégnés répondant à la formule générale

$E_I (E^x_{Ix})_y M_n$

dans laquelle :

- y = 0 ou 1,
- E et $E^x$ représentent les symboles chimiques d'un élément imprégné,
- I et $I_x$ représentent le nombre de moles d'élément mises en jeu lors de l'imprégnation ($\times 10^{-4}$ et par gramme de support),
- M représente le matériau mésoporeux support,
- n représente le nombre d'atomes de carbone de la chaîne alkyle du tensioactif utilisé dans la préparation du matériau mésoporeux,

tels que les catalyseurs mésoporeux choisis parmi les suivants :
$Li_{25} M_{16}$, $La_{25} M_{16}$, $Cs_{25} M_{16}$, $Na_{25} M_{16}$, $Mg_{25} M_{16}$, $Cs_{100} M_{16}$, $Cs_{50} M_{16}$, $Cs_6 M_{16}$, $Cs_6 La_6 M_{16}$, $Cs_{12} La_{12} M_{16}$, dans lesquels le matériau mésoporeux M est choisi parmi ceux contenant du silicium et/ou de l'aluminium.

**7.** Utilisation selon l'une des revendications 1 à 5, de catalyseurs mésoporeux imprégnés répondant à la formule générale suivante :

$E_I (E^x_{Ix})_y M_n Al_r$

dans laquelle :

- y = 0 ou 1,
- E et $E^x$ représentent les symboles chimiques d'un élément imprégné,
- I et $I_x$ représentent le nombre de moles d'élément mises en jeu lors de l'imprégnation ($\times 10^{-4}$ et par gramme de support),
- M représente le matériau mésoporeux support,
- n représente le nombre d'atomes de carbone de la chaîne alkyle du tensioactif utilisé dans la préparation du matériau mésoporeux,
- r représente le rapport molaire Si/Al, à partir des quantités d'aluminium introduites lors du mélange des réactifs lors de l'étape d'imprégnation, dans le cas où M représente un aluminosilicate mésoporeux,

tels que les catalyseurs mésoporeux choisis parmi les suivants :
$Li_{25} M_{16} Al_{20}$, $La_{25} M_{16} Al_{20}$, $Cs_{25} M_{16} Al_{20}$, $Na_{25} M_{16} Al_{20}$, $Mg_{25} M_{16} Al_{20}$, $Cs_{100} M_{16} Al_{20}$, $Cs_{50} M_{16} Al_{20}$, $Cs_6 M_{16} Al_{20}$, $Cs_6 La_6 M_{16} Al_{20}$, $Cs_{12} La_{12} M_{16} Al_{20}$, dans lesquels le matériau mésoporeux M contient du silicium et de l'aluminium.

**8.** Utilisation selon l'une des revendications 1 à 5, de catalyseurs mésoporeux greffés répondant à la formule générale suivante :

$E_{Ig} M_n$

dans laquelle :

- E représente le symbole chimique d'un élément greffé,
- I représente le nombre de moles d'élément mises en jeu lors du greffage ($\times 10^{-4}$ et par gramme de support),
- g signifie que l'élément est greffé,
- M représente le matériau mésoporeux support,
- n représente le nombre d'atomes de carbone de la chaîne alkyle du tensioactif utilisé dans la préparation du matériau mésoporeux,

tels que les catalyseurs mésoporeux choisis parmi les suivants :
$Cs_{Ig}[Si\text{-}M_{16}]$, $Al_{Ig}[Si\text{-}M_{16}]$, dans lesquels $Si\text{-}M_{16}$ signifie que le matériau mésoporeux ne contient que du silicium.

**9.** Utilisation selon l'une des revendications 1 à 5, de catalyseurs mésoporeux échangés répondant à la formule générale suivante :

$E_{le} M_n$

dans laquelle :

- E représente le symbole chimique d'un élément échangé,
- I représente le nombre de moles d'élément mises en jeu lors de l'échange ($\times 10^{-4}$ et par gramme de support),
- e signifie que l'élément est échangé,
- M représente le matériau mésoporeux support,
- n représente le nombre d'atomes de carbone de la chaîne alkyle du tensioactif utilisé dans la préparation du matériau mésoporeux,

tels que les catalyseurs mésoporeux choisis parmi les suivants :
$Cs_{le}[Si-M_{16}]$, dans lesquels $Si-M_{16}$ signifie que le matériau mésoporeux ne contient que du silicium.

**10.** Utilisation selon l'une des revendications 1 à 9, de catalyseurs mésoporeux basiques modifiés par imprégnation d'un ou plusieurs éléments, et/ou par greffage d'un ou plusieurs éléments, et/ou par échange d'un ou plusieurs éléments, tels que les catalyseurs mésoporeux échangés et greffés de formule $Cs_{le}Al_g[Si-M_{16}]$, dans laquelle $Si-M_{16}$ signifie que le matériau mésoporeux contient que du silicium, I, e, et g étant tels que définis dans les revendications 8 et 9.

**11.** Procédé d'éthérification par polymérisation du glycérol ou du glycidol, **caractérisé en ce qu'**il comprend la mise en présence de glycérol, de glycérine, ou de glycidol, avec un catalyseur solide mésoporeux basique modifié tel que défini dans l'une des revendications 1 à 10.

**12.** Procédé selon la revendication 11, **caractérisé en ce que** la quantité de catalyseur utilisée est comprise entre environ 0,001% et environ 50% en poids par rapport au poids de glycérol, glycérine ou glycidol de départ.

**13.** Procédé selon la revendication 11 ou la revendication 12, **caractérisé en ce que** la température de réaction est comprise entre environ 100°C et 300°C, notamment entre environ 150°C et environ 250°C, et **en ce qu'**il est effectué sous pression atmosphérique, ou sous pression réduite, notamment jusqu'à environ $7.10^{-3}$ bar, ou sous pression supérieure à la pression atmosphérique, notamment jusqu'à 50 bar, dans l'air ambiant ou sous atmosphère contrôlée.

**14.** Application du procédé selon l'une des revendications 11 à 13, à l'obtention de diglycérol et/ou de triglycérol, ledit procédé comprenant, le cas échéant, une étape supplémentaire de séparation d'une part du diglycérol et/ou du triglycérol, et d'autre part du glycérol restant et éventuellement des autres polyglycérols formés au cours de la réaction.

**15.** Catalyseurs mésoporeux suivants selon l'une des revendications 6 ou 7:
$Li_{25} M_{16}$, $La_{25} M_{16}$, $Cs_{25} M_{16}$, $Na_{25} M_{16}$, $Mg_{25} M_{16}$, $Cs_{100} M_{16}$, $Cs_{50} M_{16}$, $Cs_6 M_{16}$, $Cs_6 La_6 M_{16}$, $Cs_{12} La_{12} M_{16}$, $Li_{25} M_{16} Al_{20}$, $La_{25} M_{16} Al_{20}$, $Cs_{25} M_{16} Al_{20}$, $Na_{25} M_{16} Al_{20}$, $Mg_{25} M_{16} Al_{20}$, $Cs_{100} M_{16} Al_{20}$, $Cs_{50} M_{16} Al_{20}$, $Cs_6 M_{16} Al_{20}$, $Cs_6 La_6 M_{16} Al_{20}$, $Cs_{12} La_{12} M_{16} Al_{20}$.

**16.** Catalyseurs mésoporeux selon la revendication 15, dans lesquels le matériau mésoporeux M est choisi parmi ceux contenant du silicium et/ou de l'aluminium.

## Claims

**1.** Use of solid catalysts of basic mesoporous type modified by impregnation, exchange or grafting of basic elements, for the implementation of a method for the etherification by polymerisation of glycerol or glycidol, said impregnated, exchanged or grafted basic elements being chosen from the alkali elements, and/or the alkaline earth elements, and/or the rare earth elements.

**2.** Use according to claim 1, **characterised in that** the modified basic mesoporous catalysts are not soluble in glycerol or glycidol, namely in the reaction medium they do not salt out the impregnated, exchanged or grafted basic elements, such that the catalysts remain heterogeneous, are recoverable by simple filtration and are recyclable.

**3.** Use according to one of claims 1 or 2, **characterised in that** the mesoporous catalysts have pores, the size of

which is comprised between approximately 15 Å and approximately 100 Å, and preferably between approximately 15 Å and approximately 50 Å.

**4.** Use according to one of claims 1 to 3, of modified mesoporous catalysts comprising in addition to the constitutive elements of said catalysts, elements chosen from among:

- the alkali elements such as lithium, sodium, potassium or caesium;
- and/or the alkaline earths, such as magnesium, calcium or barium;
- and/or the rare earths, such as lanthanum or samarium,

the quantity of the alkali elements and/or alkaline earth elements, and/or the rare earth elements can reach up to approximately 50% by weight with respect to the total weight of the catalyst, and is, in particular, comprised between approximately 10% and approximately 20%.

**5.** Use according to one of claims 1 to 4, of mesoporous catalysts such as those obtained by implementation of a method comprising the following stages:

- addition of a solution containing the constitutive element or elements of the mesoporous material, to a solution containing a surfactant of which the number of carbon atoms of the alkyl chain is comprised between approximately 10 and approximately 20, and is preferably 16, in particular, hexadecyltrimethylammonium bromide, and if appropriate, a solution containing a salt of one or more elements to be incorporated;
- heating the gel obtained in the previous stage at approximately 100°C for approximately 24 hours;
- calcination of the solid obtained in the previous stage at a final temperature of approximately 200°C to approximately 800°C, preferably approximately 400°C to approximately 600°C, in particular approximately 550°C, advantageously for approximately 12 hours, the said final temperature being reached in successive steps of approximately 0.1°C $min^{-1}$ to approximately 20°C $min^{-1}$, starting from ambient temperature; the said calcination being advantageously carried out under an atmosphere comprising a few ppm up to 100% oxygen, preferably under diluted air comprising approximately 1% to approximately 20% oxygen, and optionally other gases such as nitrogen, helium or argon, which leads to a mesoporous catalyst of the formula $M_n$ being obtained, in which M represents the mesoporous support, and n represents the number of carbon atoms in the alkyl chain of the surfactant mentioned above, or a mesoporous material of formula $M_n\ E_r\ (E^x{}_{rx})_y$ is obtained where y = 0 or 1, E and $E^x$ represent the chemical symbols of an incorporated element, r and $r_x$ represent the molar ratio between the mesoporous material and the incorporated element (from the quantities introduced when these reactants were mixed), and M and n are as defined above;
- modification of the mesoporous catalyst obtained in the previous stage, of formula $M_n$, or formula $M_n\ E_r\ (E^x{}_{rx})_y$, such that it comprises the elements chosen from the alkali elements, and/or the alkaline earth elements, and/or the rare earth elements.

**6.** Use according to one of claims 1 to 5 of impregnated mesoporous catalysts corresponding to the general formula

$$E_I\ (E^x{}_{Ix})_y\ M_n$$

in which:

- y = 0 or 1;
- E and $E^x$ represent the chemical symbols of an impregnated element;
- I and $I_x$ represent the number of moles of an element that are involved during the impregnation (x $10^{-4}$ and per gram of support);
- M represents the mesoporous support material;
- n represents the number of carbon atoms of the alkyl chain of the surfactant used in the preparation of the mesoporous material;

such as the mesoporous catalysts chosen from the following:
$Li_{25}M_{16}$, $La_{25}M_{16}$, $Cs_{25}M_{16}$, $Na_{25}M_{16}$, $Mg_{25}M_{16}$, $Cs_{100}M_{16}$, $Cs_{50}M_{16}$, $Cs_6M_{16}$, $Cs_6La_6M_{16}$, $Cs_{12}La_{12}M_{16}$, in which the mesoporous material M is chosen from those containing silicon and/or aluminium.

**7.** Use according to one of claims 1 to 5, of impregnated mesoporous catalysts corresponding to the following general formula:

$E_l(E^x{}_{lx})_y\ M_n\ Al_r$

in which:

- y = 0 or 1;
- E and $E^x$ represent the chemical symbols of an impregnated element;
- l and $l_x$ represent the number of moles of the element involved during the impregnation (x $10^{-4}$ and per gram of support);
- M represents the mesoporous support material,
- n represents the number of carbon atoms of the alkyl chain of the surfactant used in the preparation of the mesoporous material;
- r represents the molar ratio Si/Al, from the quantities of aluminium introduced during mixing of the reagents during the impregnation stage, in the case where M represents a mesoporous aluminosilicate;

such as the mesoporous catalysts chosen from the following:
$Li_{25}M_{16}Al_{20}$, $La_{25}M_{16}Al_{20}$, $Cs_{25}M_{16}Al_{20}$, $Na_{25}M_{16}Al_{20}$, $Mg_{25}M_{16}Al_{20}$, $Cs_{100}M_{16}Al_{20}$, $Cs_{50}M_{16}Al_{20}$, $Cs_6M_{16}Al_{20}$, $Cs_6La_6M_{16}Al_{20}$, $Cs_{12}La_{12}M_{16}Al_{20}$, where the mesoporous material M contains silicon and aluminium.

**8.** Use according to one of claims 1 to 5, of grafted mesoporous catalysts corresponding to the following general formula:

$E_{lg}\ M_n$

in which:

- E represents the chemical symbol of a grafted element;
- l represents the number of moles of the element involved during grafting (x $10^{-4}$ and per gram of support);
- g signifies that the element is grafted;
- M represents the mesoporous support material;
- n represents the number of carbon atoms of the alkyl chain of the surfactant used in the preparation of the mesoporous material;

such as the mesoporous catalysts chosen from the following:
$Cs_{lg}$[Si-$M_{16}$], $Al_{lg}$[Si-$M_{16}$], in which Si-$M_{16}$ signifies that the mesoporous material contains silicon only.

**9.** Use according to one of claims 1 to 5, of exchanged mesoporous catalysts corresponding to the following general formula:

$E_{le}\ M_n$

in which:

- E represents the chemical symbol of an exchanged element;
- l represents the number of moles of the element involved during the exchange (x $10^{-4}$ and per gram of support);
- e signifies that the element is exchanged;
- M represents the mesoporous support material;
- n represents the number of carbon atoms of the alkyl chain of the surfactant used in the preparation of the mesoporous material;

such as the mesoporous catalysts chosen from the following:
$Cs_{le}$[Si-$M_{16}$], where Si-$M_{16}$ signifies that the mesoporous material contains silicon only.

**10.** Use according to one of claims 1 to 9 of basic mesoporous catalysts modified by impregnation of one or more elements, and/or by grafting of one or more elements, and/or by exchange of one or more elements, such that the exchanged and grafted mesoporous catalysts of formula $Cs_{le}Al_g$[Si-$M_{16}$], in which Si-$M_{16}$ signifies that the mesoporous material contains only silicon, l, e and g being as defined in claims 8 and 9.

**11.** Method for the etherification by polymerisation of glycerol or glycidol, **characterised in that** it comprises the introduction of glycerol, glycerine or glycidol into a basic mesoporous solid catalyst modified as defined in one of claims 1 to 10.

**12.** Method according to claim 11, **characterised in that** the quantity of catalyst used is comprised between approximately 0.001% and approximately 50% by weight with respect to the weight of glycerol, glycerine or glycidol at the start.

**13.** Method according to claim 11 or claim 12, **characterised in that** the reaction temperature is comprised between approximately 100°C and 300°C, in particular between approximately 150°C and approximately 250°C, and **in that** it is carried out under atmospheric pressure, or under reduced pressure, in particular up to approximately $7.10^{-3}$ bar, or under pressure greater than atmospheric pressure, in particular up to 50 bar, in ambient air or under a controlled atmosphere.

**14.** Application of the method according to one of claims 11 to 13, for obtaining diglycerol and/or triglycerol, said method comprising, if appropriate, an additional stage of separation on the one hand of the diglycerol and/or the triglycerol, and on the other hand of the remaining glycerol and optionally other polyglycerols formed during the reaction.

**15.** The following mesoporous catalysts according to one of claims 6 or 7:
$Li_{25}M_{16}$, $La_{25}M_{16}$, $Cs_{25}M_{16}$, $Na_{25}M_{16}$, $Mg_{25}M_{16}$, $Cs_{100}M_{16}$, $Cs_{50}M_{16}$, $Cs_6M_{16}$, $Cs_6La_6M_{16}$, $Cs_{12}La_{12}M_{16}$, $Li_{25}M_{16}Al_{20}$, $La_{25}M_{16}Al_{20}$, $Cs_{25}M_{16}Al_{20}$, $Na_{25}M_{16}Al_{20}$, $Mg_{25}M_{16}Al_{20}$, $Cs_{100}M_{16}Al_{20}$, $Cs_{50}M_{16}Al_{20}$, $Cs_6M_{16}Al_{20}$, $Cs_6La_6M_{16}Al_{20}$, $Cs_{12}La_{12}M_{16}Al_{20}$.

**16.** Mesoporous catalysts according to claim 15, in which the mesoporous material M is chosen from those containing silicon and/or aluminium.

**Patentansprüche**

**1.** Verwendung von basischen mesoporösen festen Katalysatoren, die durch Imprägnierung, Austausch oder Pfropfung basischer Elemente modifiziert sind, für die Umsetzung eines Verfahrens der Veretherung von Glycerol oder Glycidol durch Polymerisation, wobei die imprägnierten, getauschten oder gepfropften basischen Elemente aus den Alkalielementen und/oder Erdalkalielementen und/oder Seltenen Erden ausgewählt sind.

**2.** Verwendung gemäß Anspruch 1, **dadurch gekennzeichnet, dass** die modifizierten basischen mesoporösen Katalysatoren in dem Glycerol oder Glycidol nicht löslich sind, dass sie nämlich im Reaktionsmilieu die imprägnierten, getauschten oder gepfropften basischen Elemente nicht aussalzen, so dass die Katalysatoren heterogen bleiben, durch einfache Filtration wiedergewonnen werden können und recyclebar sind.

**3.** Verwendung gemäß einem der Ansprüche 1 oder 2, **dadurch gekennzeichnet, dass** die mesoporösen Katalysatoren Poren haben, deren Größe zwischen etwa 15 Å und etwa 100 Å und vorzugsweise zwischen etwa 15 Å und etwa 50 Å liegt.

**4.** Verwendung gemäß einem der Ansprüche 1 bis 3 von modifizierten mesoporösen Katalysatoren, die zusätzlich zu den konstituierenden Elementen der Katalysatoren Elemente enthalten, die ausgewählt sind aus:

- Alkalielementen, wie Lithium, Natrium, Kalium oder Cäsium,
- und/oder Erdalkalielementen, wie Magnesium, Calcium oder Barium,
- und/oder Seltenen Erden, wie Lanthan oder Samarium,

wobei die Menge der Alkalielemente und/oder Erdalkalielemente und/oder Seltenen Erden bis zu etwa 50 Gew.-% im Verhältnis zum Gesamtgewicht des Katalysators erreichen kann und insbesondere zwischen etwa 10 und etwa 20 % beträgt.

**5.** Verwendung gemäß einem der Ansprüche 1 bis 4 von mesoporösen Katalysatoren, wie sie durch Umsetzung eines Verfahrens erhalten werden, das folgende Schritte umfasst:

- Zugabe einer Lösung, die das oder die konstituierenden Elemente des mesoporösen Materials enthält, zu einer Lösung, die ein Tensid enthält, bei dem die Zahl an Kohlenstoffatomen der Alkylkette zwischen etwa 10

und etwa 20 liegt und vorzugsweise 16 ist, insbesondere Hexadecyltrimethylammoniumbromid, und gegebenenfalls einer Lösung, die ein Salz eines oder mehrerer einzubringender Elemente enthält,
- Erhitzen des im vorangegangenen Schritt erhaltenen Gels auf etwa 100 °C für etwa 24 Stunden,
- Calcinieren des im vorangegangen Schritt erhaltenen Feststoffs bei einer Endtemperatur von etwa 200 °C bis etwa 800 °C, vorzugsweise etwa 400 °C bis etwa 600 °C, insbesondere etwa 550 °C, geeigneterweise für etwa 12 Stunden, wobei die Endtemperatur durch aufeinander folgende Phasen von etwa 0,1 $°C.min^{-1}$ bis etwa 20 $°C.min^{-1}$ ausgehend von Umgebungstemperatur erreicht wird, wobei das Calcinieren geeigneterweise unter Atmosphäre mit einigen ppm bis zu 100 % Sauerstoff, vorzugsweise unter verdünnter Luft mit etwa 1 bis etwa 20 % Sauerstoff und gegebenenfalls anderen Gasen, wie Stickstoff, Helium oder Argon, bewirkt wird, was zur Gewinnung eines mesoporösen Katalysators der Formel $M_n$, worin M das mesoporöse Trägermaterial und n die Zahl der Kohlenstoffatome der Alkylkette des oben genannten Tensids darstellt, oder zur Gewinnung eines mesoporösen Materials der Formel $M_nE_r(E^x{}_{rx})_y$, worin y = 0 oder 1, E und $E^x$ die chemischen Symbole eines eingebrachten Elements darstellen, r und $r_x$ das Molverhältnis zwischen dem mesoporösen Material und dem eingebrachten Element (ausgehend von den beim Mischen der Reagenzien eingebrachten Mengen) darstellen und M und n wie oben definiert sind, führt,
- Modifizieren des im vorangegangenen Schritt erhaltenen mesoporösen Katalysators der Formel $M_n$ oder der Formel $M_nE_r(E^x{}_{rx})_y$ derart, dass er Elemente enthält, die aus den Alkalielementen und/oder Erdalkalielementen und/oder Seltenen Erden ausgewählt sind.

**6.** Verwendung gemäß einem der Ansprüche 1 bis 5 von imprägnierten mesoporösen Katalysatoren, die der allgemeinen Formel

$$E_l(E^x{}_{lx})_yM_n$$

entsprechen, worin:

- y = 0 oder 1,
- E und $E^x$ die chemischen Symbole eines imprägnierten Elements darstellen,
- l und $l_x$ die Zahl der Mole des Elements darstellen, die beim Imprägnieren ins Spiel gebracht werden (x $10^{-4}$ und pro Gramm Träger),
- M das mesoporöse Trägermaterial darstellt,
- n die Zahl der Kohlenstoffatome der Alkylkette des bei der Herstellung des mesoporösen Materials verwendeten Tensids darstellt,

wie den mesoporösen Katalysatoren, die aus folgenden ausgewählt sind:
$Li_{25}M_{16}$, $La_{25}M_{16}$, $Cs_{25}M_{16}$, $Na_{25}M_{16}$, $Mg_{25}M_{16}$, $Cs_{100}M_{16}$, $Cs_{50}M_{16}$, $Cs_6M_{16}$, $Cs_6La_6M_{16}$, $Cs_{12}La_{12}M_{16}$, in welchen das mesoporöse Material M aus jenen ausgewählt ist, die Silicium und/oder Aluminium enthalten.

**7.** Verwendung gemäß einem der Ansprüche 1 bis 5 von imprägnierten mesoporösen Katalysatoren, die der folgenden allgemeinen Formel entsprechen:

$$E_l(E^x{}_{lx})_yM_nAl_r$$

worin:

- y = 0 oder 1,
- E und $E^x$ die chemischen Symbole eines imprägnierten Elements darstellen,
- l und $l_x$ die Zahl der Mole des Elements darstellen, die beim Imprägnieren ins Spiel gebracht werden (x $10^{-4}$ und pro Gramm Träger),
- M das mesoporöse Trägermaterial darstellt,
- n die Zahl der Kohlenstoffatome der Alkylkette des bei der Herstellung des mesoporösen Materials verwendeten Tensids darstellt,
- r das Molverhältnis Si/Al darstellt, ausgehend von den Aluminiummengen, die beim Mischen der Reagenzien beim Imprägnierungsschritt eingebracht wurden, in dem Fall, wo M ein mesoporöses Aluminiumsilicat darstellt,

wie den mesoporösen Katalysatoren, die aus folgenden ausgewählt sind:
$Li_{25}M_{16}Al_{20}$, $La_{25}M_{16}Al_{20}$, $Cs_{25}M_{16}Al_{20}$, $Na_{25}M_{16}Al_{20}$, $Mg_{25}M_{16}Al_{20}$, $Cs_{100}M_{16}Al_{20}$, $Cs_{50}M_{16}Al_{20}$, $Cs_6M_{16}Al_{20}$, $Cs_6La_6M_{16}Al_{20}$, $Cs_{12}La_{12}M_{16}Al_{20}$, bei welchen das mesoporöse Material M Silicium und Aluminium enthält.

**8.** Verwendung gemäß einem der Ansprüche 1 bis 5 von gepfropften mesoporösen Katalysatoren, die der folgenden allgemeinen Formel entsprechen:

$$E_{Ig}M_n$$

worin:

- E das chemische Symbol eines gepfropften Elements darstellt,
- I die Zahl der Mole des Elementes darstellt, die beim Pfropfen ins Spiel gebracht werden (x $10^{-4}$ und pro Gramm Träger),
- g anzeigt, dass das Element gepfropft ist,
- M das mesoporöse Trägermaterial darstellt,
- n die Zahl der Kohlenstoffatome der Alkylkette des bei der Herstellung des mesoporösen Materials verwendeten Tensids darstellt,

wie den mesoporösen Katalysatoren, die aus folgenden ausgewählt sind:
$Cs_{Ig}$[Si-$M_{16}$], $Al_{Ig}$[Si-$M_{16}$], bei welchen Si-$M_{16}$ bedeutet, dass das mesoporöse Material nur Silicium enthält.

**9.** Verwendung gemäß einem der Ansprüche 1 bis 5 von ausgetauschten mesoporösen Katalysatoren, die der folgenden allgemeinen Formel entsprechen:

$$E_{Ie}M_n$$

worin:

- E das chemische Symbol eines getauschten Elements darstellt,
- I die Zahl der Mole des Elements darstellt, die beim Austausch ins Spiel gebracht werden (x $10^{-4}$ und pro Gramm Träger),
- e anzeigt, dass das Element getauscht ist,
- M das mesoporöse Trägermaterial darstellt,
- n die Zahl der Kohlenstoffatome der Alkylkette des bei der Herstellung des mesoporösen Materials verwendeten Tensids darstellt,

wie den mesoporösen Katalysatoren, die aus folgenden ausgewählt sind:
$Cs_{Ie}$[Si-$M_{16}$], in welchen Si-$M_{16}$ bedeutet, dass das mesoporöse Material nur Silicium enthält.

**10.** Verwendung gemäß einem der Ansprüche 1 bis 9 von basischen mesoporösen Katalysatoren, die durch Imprägnieren mit einem oder mehreren Elementen und/oder durch Pfropfung von einem oder mehreren Elementen und/oder durch Austausch von einem oder mehreren Elementen modifiziert sind, wie den getauschten und gepfropften mesoporösen Katalysatoren der Formel $Cs_{Ie}Al_g$[Si-$M_{16}$], worin Si-$M_{16}$ bedeutet, dass das mesoporöse Material nur Silicium enthält, und I, e und g wie in den Ansprüchen 8 und 9 definiert sind.

**11.** Verfahren der Veretherung von Glycerol oder Glycidol durch Polymerisation, **dadurch gekennzeichnet, dass** es das Zusammenbringen von Glycerol, Glycerin oder Glycidol mit einem basischen mesoporösen festen Katalysator umfasst, der, wie in einem der Ansprüche 1 bis 10 definiert, modifiziert ist.

**12.** Verfahren gemäß Anspruch 11, **dadurch gekennzeichnet, dass** die verwendete Katalysatormenge zwischen etwa 0,001 und etwa 50 Gew.-% in Bezug auf das Ausgangsgewicht des Glycerols, des Glycerins oder des Glycidols liegt.

**13.** Verfahren gemäß Anspruch 11 oder Anspruch 12, **dadurch gekennzeichnet, dass** die Reaktionstemperatur zwischen etwa 100 °C und 300 °C, insbesondere zwischen etwa 150 °C und etwa 250 °C, liegt und dass es unter atmosphärischem Druck oder reduziertem Druck, insbesondere bis etwa $7.10^{-3}$ bar oder unter über atmosphärischen Druck erhöhtem Druck, insbesondere bis 50 bar, in Umgebungsluft oder unter kontrollierter Atmosphäre durchgeführt wird.

**14.** Anwendung des Verfahrens gemäß einem der Ansprüche 11 bis 13, um Diglycerol und/oder Triglycerol zu erhalten, wobei das Verfahren gegebenenfalls einen zusätzlichen Schritt der Trennung von einerseits Diglycerol und/oder Triglycerol und andererseits verbleibendem Glycerol sowie gegebenenfalls anderen im Laufe der Reaktion gebildeten Polyglycerolen enthält.

**15.** Mesoporöse Katalysatoren gemäß einem der Ansprüche 6 oder 7 wie folgt: $Li_{25}M_{16}$, $La_{25}M_{16}$, $Cs_{25}M_{16}$, $Na_{25}M_{16}$, $Mg_{25}M_{16}$, $Cs_{100}M_{16}$, $Cs_{50}M_{16}$, $Cs_6M_{16}$, $Cs_6La_6M_{16}$, $Cs_{12}La_{12}M_{16}$, $Li_{25}M_{16}Al_{20}$, $La_{25}M_{16}Al_{20}$, $Cs_{25}M_{16}Al_{20}$, $Na_{25}M_{16}Al_{20}$, $Mg_{25}M_{16}Al_{20}$, $Cs_{100}M_{16}Al_{20}$, $Cs_{50}M_{16}Al_{20}$, $Cs_6M_{16}Al_{20}$, $Cs_6La_6M_{16}Al_{20}$, $Cs_{12}La_{12}M_{16}Al_{20}$.

**16.** Mesoporöse Katalysatoren gemäß Anspruch 15, bei denen das mesoporöse Material M aus jenen ausgewählt ist, die Silicium und/oder Aluminium enthalten.